# EUROPEAN PATENT SPECIFICATION

(11) **EP 0 372 091 B1**
(45) Date of publication and mention of the grant of the patent: **18.09.1996**
(21) Application number: 89907265.6
(22) Date of filing: 09.06.1989
(51) Int. Cl.: A23L 3/3526, A23L 3/37, A23L 3/3571, A23L 3/3418

(54) **METHOD FOR PRESERVING FOOD**
VERFAHREN ZUR KONSERVIERUNG VON NAHRUNGSMITTELN
PROCEDE DE CONSERVATION D'ALIMENTS

(30) Priority: 09.06.1988 JP 144122/88
(43) Date of publication of application: 13.06.1990
(73) Proprietor: Kabushiki Kaisha Ueno Seiyaku Oyo Kenkyujo, Osaka-shi Osaka-fu (JP)
(72) Inventor: UENO, Ryuzo, Nishinomiya-shi Hyogo 662 (JP); FUJITA, Yatsuka, Nishinomiya-shi Hyogo 662 (JP); NAGAMURA, Yoshiaki, Ushiku-cho Ushiku-shi Ibaraki 300-12 (JP); KAMINO, Yuji, Tsukuba-shi, Ibaraki 305 (JP); TABATA, Akihiko, Ushiku-shi Ibaraki 300-12 (JP)
(74) Representative: Atkinson, Peter Birch
(86) International application number: JP8900587
(87) International publication number: WO8911801

(56) References cited:
- EP-A- 0 265 884
- US-A- 4 619 995
- CHEMICAL PATENTS INDEX, BASIC ABSTRACTS JOURNAL Derwent Publications Ltd., London, GB; AN 88-061223
- CHEMICAL PATENTS INDEX, BASIC ABSTRACTS JOURNAL Derwent Publications Ltd., London, GB; AN 89-125909
- PATENT ABSTRACTS OF JAPAN vol. 12, no. 76 (C-473)4 February 1988 ( NIPPON KAYAKU CO. ET AL. )
- CHEMICAL PATENTS INDEX, BASIC ABSTRACTS JOURNAL Derwent Publications Ltd., London, GB; AN 84-143209
- CHEMICAL PATENTS INDEX, BASIC ABSTRACTS JOURNAL Derwent Publications Ltd., London, GB; AN 86-241855
- CHEMICAL PATENTS INDEX, BASIC ABSTRACTS JOURNAL Derwent Publications Ltd., London, GB; AN 87-217613
- Japan Food Science, Vol. 22, No. 5, May 1983 (TOkyo), Toyoda Takuo (Datsu Sansozai Riyo ni yoru Shokuhin no Hozon Gijutsu) pp. 36-43
- Japan Food Science, Vol. 27, No. 5, May 1988 (Tokyo), Kodaka Akira, Nosaka Noriyoshi (Tennenbutsu Riyo ni yoru Shokuhin Hozonryo Kaihatsu no Genkyo) pp. 25-34

## Description

### TECHNICAL FIELD

The present invention relates to a preservation of foods.

### BACKGROUND ART

It has been well known that an antimicrobial agent is added to the foods for the preservation. Natural antimicrobial agents such as protamine, chitosan, lysozyme and the like have been used as an antimicrobial agent, but these natural antimicrobial agents are limited in the amount to be added to foods, and are not satisfactorily efficacious in the antimicrobial activity against some kinds of microbe or yeast fungi and so on.

On the other hand, it has been known that the method of controlling oxygen concentration lower by deoxydizing agents or gas substitution is effective to preserve foods, for example, Japanese confections, Western-style cakes, breads, noudles, dry saltery and so forth. These methods are also insufficient to prevent foods from the attack of some kinds of microbe, yeast or fungi and the like. Therefore, the independent preservation methods of foods by use of a natural antimicrobial agent or by the reduction of oxygen gas content using a deoxydizing agent or gas substitution have not led to a satisfactory result because of their insufficient antimicrobial activity against some kinds of microbe, yeast or fungi.

Natural antimicrobial agents, which themselves are used by directly spraying, dipping or mixing into foods are limited in the amount to be used, because they influence delicately the sense of taste and smell if they are used in much amount.

Japanese patent specification no. JP-A-63 017 679 (Chemical Patents Index A.N. 88-061223) discloses a preservative agent for food which contains protamin and ascorbic acid together with an amino acid and glutathione, which are added directly into the food.

Japanese patent specification no. JP-A-62 143 672 (Chemical Patents Index A.N. 87-217613) discloses a preservative agent for food which contains lysozyne and glucose or ascorbic acid together with glucose oxidase, which are again added directly into the food to be preserved.

Japanese patent specification no. JP-A-63 186 942 (Patent Extracts of Japan, 12, no. 76(C-473)4) discloses a method for preserving food using a deoxidising agent which contains an iron compound and chitosan packed together in the a gas permeable bag which is stored with the food to be preserved. The chitosan is included to reduce the amount of an aldehyde which is generated through the combined use of ethyl alcohol and iron in the deoxidising agent.

The object of the present invention is to provide a method of preserving foods, which is broadly effective to prevent foods from deterioration and from denaturation caused by microbe, yeast and/or fungi even in the use of small amount of antimicrobial agents.

According to the present invention there is provided a method of preserving food, comprising adding to food at least one kind of a natural antimicrobial agent selected from the group consisting of protamin, chitosan and lysozyme, and storing the food in the presence of a deoxidising agent, wherein the food containing said natural antimicrobial agent is sealed together with the deoxidising agent in the same sealed package.

The method of the present invention can be applied to various kinds of foods, for instance, Japanese confections, such as namagashi (soft and fresh Japanese sweets), yokan (sweet jelly of beans), monaka (wafer cake stuffed with bean jam), uirou (rice jelly), habutaemochi (soft rice cake) and the like; Western style cakes such as sponge cake, crepe, pie, fresh cream, custard cream and the like; staple foods such as bread, rice, fresh noodles, rice cake, shiratama (rice-flour dumpling), oshizushi (pressed sushi) and the like; dry salteries or chinmi (gourmet) such as surume (dried squid), niboshi (cooked and dried fish), kunsei (smoking), denbu (tiny bits of fish boiled with sugar and soy sauce), katsuobushi (dried bonito), kanpyo (dried gourd shavings), dried shiitake mushroom, dried vegetable, dried fruit and the like; fish or meat processed materials such as kamaboko (boiled fish paste), chikuwa (tube-shaped fish paste), cheese, ham, sausage; an agricultural processed material such as pickles, fruits soaked into sugar, jam and the like; perishable foods such as vegetables, fruits, fish, meats and the like; seasonings such as miso (fermented soybean paste), mentsuyu (soup stock for noodle), liquid seasonings, powder seasonings, furikake (tasty seasoned and dried food for sprinkling over rice), and the like; seasoned foods, sozai (side dish) and the like. A remarkable effect is observed when the method of the present invention is applied to foods of high water content, especially to foods having a water activity more than 0.8, which are difficult to be preserved for a long time by the conventional way.

Examples of a natural antimicrobial agent having an amino group used in the present invention are selected from protamine, chitosan and lysozyme. Co-use of these natural antimicrobial agents gives, of course, a preferable result. Protamine can be used in free state, and mineral acid salts of protamine such as sulfate, hydrochloride and the like may be used. When foods added with any of three kinds of natural antimicrobial agent are stored under substantially sealed condition in the presence of deoxydizing agents, the excellent preservation effect can be achieved.

The natural antimicrobial agent may be used in such an amount that the flavor and tasty of the foods are not spoiled. The amounts are 10 - 50,000 ppm, preferably 100 - 2,000 ppm for protamine, 1 - 10,000 ppm, preferably 5 - 500 ppm for lysozyme, and 5 - 50,000 ppm, preferably 10 - 1,000 ppm for chitosan. These antimicrobial agents may be used singly or mixed.

The natural antimicrobial agent may be added to food materials before cooking, or may be directly added to foods and simply mixed. Alternatively, a solution of the natural antimicrobial agent in suitable solvent such as ethanol or water may be sprayed or sprinkled on foods. And foods can be soaked in the solution.

Foods to which the natural antimicrobial agent is added are sealed together with a deoxydizing agent, and preferably stored in oxygen free circumstances or lower oxygen concentration, for instance, not more than 0.05 %, preferably not more than 0.01 %, by maintaining the foods in substantially sealed condition.

As deoxydizing agents, an arbitrary mixture can be used which is made from mainly several reducing agents such as, for example, sulfites, hydrogensulfites, thiosulfates, dithionites, oxalates, pyrogallol, Rongalit, glucose, copper-amine complexes, ascorbic acid, iron powders, zinc powders.

The deoxydizing agent of the present invention may be used in such an amount that the concentration of oxygen in a sealed package can be maintained less than about 0.05 %, preferably less than about 0.01 %. Generally, it is preferable to use it in excess amount so as to remove oxygen which permeates continuously into the sealed package through a wrapping film with time.

A wrapping material may be anything having the lower oxygen permeability, for example, vinylidenechloride-coated laminate film, a can, a bottle, a jar and the like which can be shut airtightly, though not restrictively.

Any other antimicrobial agent may be used together with the natural antimicrobial agent in the present invention.

The present invention is illustrated by the following Examples.

### EXAMPLE 1 and COMPARATIVE EXAMPLE 1:

A standard agar plate culture medium (pH 7.0) containing protamine chloride (available from SIGMA, from salmon) in the concentration of 62.5 - 1,000 ppm was prepared sterilely, onto which a culture solution of microorganisms (10⁶ cfu/ml) shown in Table 1 was inoculated by a microplanter (MIT-P) available from K. K. Sakuma Seisakusho. The culture medium inoculated was sealed in a bag (volume: 450 cc) of KOP/PE (20µ /50µ ) together with a deoxydizing agent Oxyta H-100 (available from Ueno Seiyaku K.K.; iron type deoxydizing agent), and cultured at 30 °C for 2 weeks. The oxygen content in each bag during the test was 0.005 - 0.009 %. The minimum concentration of protamine hydrochloride, at which growth of the microorganisms has not been completely inhibited is shown in Table 1.

In the Comparative Example 1 such antimicrobial tests were carried out in the same manner as described in the Example 1 except that no deoxydizing agent was used. The results are shown in Table 1.

In controls which were carried out according to the same manner as in Example 1 except that the protamine hydrochloride was not used, the growth of every microorganisms was observed, though the oxygen concentration was substantially the same as in Example 1.

**Table 1**

| Microorganisms | Example 1 | Comp. Ex. 1 |
|---|---|---|
| L. casei sub sp. rhamnosus IFO 3425 | 250 | 500 |
| L. fermentum IFO 3071 | 250 | 500 |
| L. fructosus IFO 3516 | 250 | 500 |
| L. lactis IFO 3734 | <62.5 | 250 |
| L. murinus IFO 14221 | <62.5 | 500 |
| L. plantarum IFO 3070 | 250 | 500 |
| Leuc. lactis IFO 12455 | 250 | 700 |
| Leuc. mesenteroides IFO 3426 | 250 | 500 |
| Leuc. mesenteroides IAM 1046 | 250 | 500 |
| Streptococcus pyogenes ATCC 19615 | 250 | 700 |
| | | (ppm) |
| L. : Lactobacillus, Leuc. : Leuconostoc | | |

### EXAMPLE 2 and COMPARATIVE EXAMPLE 2

A potato dextrose agar plate culture medium (pH 7.0) containing protamine sulfate (available from Wako Junyaku K. K., from herring) at the concentration of 62.5 - 2,000 ppm was sterilely prepared, onto which yeast culture solution (10⁶ cfu/ml) was inoculated by a microplanter (MIT-P) available from Sakuma Seisakusho. The culture medium inoculated was sealed in a bag (volume: 450 cc) of KOP/PE (20µ /50µ ) together with a deoxydizing agent Oxyta H-100 (available from Ueno Seiyaku K.K.; iron type deoxydizing agent), and cultured at 30 °C for one week. The oxygen content in each bag during the test was 0.005 - 0.009 %. The minimum concentration of protamine sulfate, at which growth of the yeasts has been completely inhibited is shown in Table 2.

In the Comparative Example 2 antimicrobial tests were carried out in the same manner as described in the Example 2 except that no deoxydizing agent was used. The results are shown in Table 2.

In controls which were carried out according to the same manner as in Example 2 except that the protamine sulfate was not used, the growth of every yeast was observed, though the oxygen concentration in each bag was substantially the same as in Example 2.

**Table 2**

| Yeast | Example 2 | Comp. Ex. 2 |
|---|---|---|
| Saccharomyces cerevisiae IAM 4272 | 1,000 | 2,000 |
| Saccharomyces cereviciae IAM 4274 | 1,000 | 2,000 |
| Saccharomyces ovif. Ost. S-73 | 1,000 | 2,000 |
| Candida utilis IFO 0396 | 500 | 1,000 |
| Cryptococcus laurentii IAM 12264 | 500 | 1,000 |
| Rhodotorula rubra IFO 0894 | 500 | 1,000 |
| Rhodotorula rubra IAM 4989 | 250 | 700 |
| Shizosaccharomyces pombe Lindner 0-77 | 700 | 2,000 |
| Torulopsis Candida IAM 4976 | 700 | 2,000 |
| Trichosporon cutaneum IAM 12246 | 500 | 1,000 |
| | | (ppm) |

### EXAMPLE 3 and COMPARATIVE EXAMPLE 3

A standard agar plate culture medium (pH 7.0) containing lysozyme (available from Seikagaku Kogyo K. K., 6-times crystallized product, from egg white) at the concentration of 1.25 - 160 ppm was sterilely prepared, onto which a microorganism culture solution (10⁶ cfu/ml) was inoculated by a microplanter (MIT-P) available from Sakuma Seisakusho. The culture medium inoculated was sealed in a bag (volume: 450 cc) of KOP/PE (20µ /50µ ) together with a deoxydizing agent Oxyta H-100 (available from Ueno Seiyaku K.K.; iron type deoxydizing agent), and cultured at 30 °C for one hour. The oxygen content in each bag during the test was 0.005 - 0.009 %. The minimum concentration of lysozyme, at which growth of the microorganisms has been completely inhibited is shown in Table 3.

In the Comparative Example 3 antimicrobial tests were carried out in the same manner as described in the Example 3 except that no deoxydizing agent was used. The results are shown in Table 3.

In controls which were carried out according to the same manner as in Example 3 except that the lysozyme was not used, the growth of every microorganisms was observed, though the oxygen concentration was substantially the same as in Example 3.

**Table 3**

| microorganism | Example 3 | Comp. Ex. 3 |
|---|---|---|
| L. casei sub sp. rhamnosus IFO 3425 | 40 | 160 |
| L. plantarum IFO 3070 | 40 | 160 |
| Leuc. lactis IFO 12455 | 40 | 160 |
| Leuc. mesenteroides IFO 3426 | 2.5 | 160 |
| Leuc. mesenteroides IFO 1046 | 40 | 160 |
| | | (ppm) |
| L.: Lactobacillus, Leuc.: Leuconostoc | | |

### EXAMPLE 4 and COMPARATIVE EXAMPLE 4

Nutrient Broth (available from Difco, pH 6.0) containing 1 % glucose, and chitosan (available from Katayama Kagaku Kogyo K.K.) at a concentration of 7.8 - 250 ppm was prepared sterilely, onto which each one loop of microorganism culture solution of Table 4 was inoculated. Each culture medium inoculated was sealed in a bag (450 cc) made of KOP/PE (20µ /50µ ) together with an deoxydizing agent, Oxyta H-100 (available from Ueno Seiyaku K.K.), cultivated at 30 °C for one week. After that the bag of cultivated medium was opened, one loop of it was inoculated onto a usual agar plate culture medium, and cultivated at 30 °C for 2 days.

The oxygen content in each bag during the test is 0.005 - 0.009 %. The minimum concentration of chitosan, at which growth of the microorganisms has been completely inhibited is shown in Table 4.

In the Comparative Example 4 such antimicrobial tests were carried out in the same manner as described in the Example 4 except that no deoxydizing agent was used. The results are shown in Table 4.

In controls which were carried out according to the same manner as in Example 4 except that the chitosan was not used, the growth of every microorganisms was observed, though the oxygen concentration was substantially the same as in Example 4.

**Table 4**

| microorganisms | Example 4 | Comp. Ex. 4 |
|---|---|---|
| L. casei sub sp. rhamnosus IFO 3425 | <7.8 | 31.3 |
| L. fermentum IFO 3071 | <7.8 | 15.6 |
| L. lactis IFO 3734 | <7.8 | 15.6 |
| L. plantarum IFO 3070 | <7.8 | 31.3 |
| Leuc. lactis IFO 12455 | 15.6 | 31.3 |
| Leuc. mesenteroides IFO 3426 | <7.8 | 62.5 |
| Leuc. mesenteroides IFO 1046 | <7.8 | 31.3 |
| Streptococcus pyogenes ATCC 19615 | <7.8 | 15.6 |
| | | (ppm) |
| L. : Lactobacillus casei sub sp. rhamnosus, Leuc. : Leuconostoc | | |

### EXAMPLE 5

### 1. PREPARATION

Hard flour (Midori Naito, Nitto Seifun K. K.) 250 g, salt (purified salt) 8 g, kansui powder (alkaline material obtained by concentrating a lye of a stem of plantain or banana, which is used for increasing the consistency of dough; a reagent) 2.4 g, coloring agent (Rikecolor Y, available from Riken Vitamin K.K.) 0.16 g and aqueous solution containing protamine chloride in a prescribed amount 144 g were mixed by a mixer, and the mixture was pressed by a noodle making machine having a three steps-rollers and then cut by a 10th grade cutter to give nama chukamen (raw chinese noodle).

### 2. TEST METHOD

Each 50 g of the nama chukamen containing protamine chloride (available from SIGMA, from herring) and one containing no protamine chloride was sealed with the deoxydizing agent (Oxyta H-100, available from Ueno Seiyaku K.K.) and without it in a bag (450 cc) made of KOP/PE (20 µ/50 µ) respectively, and the preservability of each chukamen at 30 °C was observed.

The preservability was evaluated by observing the occurrence of colony, formation of slime, softening or discoloring of the chukamen, and the like, and expressed by "effective preservable days", which means the number of days during which the above phenomena were not observed.

### 3. RESULTS

| Group | Effective Preservable Days |
|---|---|
| containing no protamine chloride | 2 |
| containing deoxydizing agent alone | 3 |
| containing protamine Chloride 0.1 % alone | 2 |
| containing protamine chloride 0.5 % alone | 3 |
| containing protamine chloride 0.1 % with deoxydizing agent | 5 |

### EXAMPLE 6

### 1. PREPARATION

Churikiko (medium flour; Akanaito, available from Nitto Seifun K.K.) 1 kg was added with 6 % aqueous salt (purified) solution containing protamine sulfate in a prescribed amount 350 g, and the mixture was stirred by a Kanto Mixer CS25E Type. The product was extended under pressure, stamped with feat, rolled, and then cut to give nama udon (raw noodle).

### 2. TEST METHOD

Each 50 g of the nama udon containing protamine sulfate (available from Wako Junyaku K.K., from herring) and one containing no protamine sulfate was sealed with deoxydizing agent (Oxyta H-100, available from Ueno Seiyaku K.K.) and without it in a bag (450 cc) made of KOP/PE (20 µ/50 µ) respectively, and the preservability of each nama udon at 30 °C was observed.

The preservability was evaluated by observing the occurrence of colony, formation of slime, softening or discoloring of the udon, and the like, and expressed by "effective preservable days", which means the number of days during which the above phenomena were not observed.

### 3. RESULT

| Group | Effective Preservable Days |
|---|---|
| containing no protamine sulfate | 1 |
| containing deoxydizing agent alone | 2 |
| containing protamine sulfate 0.1 % alone | 1 |
| containing protamine sulfate 0.5 % alone | 1.5 |
| containing protamine sulfate with deoxydizing agent | 5 |

### EXAMPLE 7

### 1. PREPARATION

Lighter-colored soy sauce 500 cc, Mirin (Sweet sake for seasoning) 500 cc, soup stock from a dried bonito and a sea tangle 3,000 cc were mixed, boiled for a short time, and then cooled to give soup for buckwheat noodles.

### 2. TEST METHOD

Each 100 cc of the soup for buckwheat noodles containing with chitosan available from Katayama Kagaku Kogyo K.K. and not containing chitosan was sealed with a deoxydizing agent (Oxyta H-100, available from Ueno Seiyaku K.K.) in a 400 cc of bottle, and the preservability at 30 °C was observed.

Each 1 ml of the soup for buckwheat noodles was used for test of preservability as one test group. The viable count of microorganisms was countered after the cultivation on a standard agar culture medium at 37 °C for 48 hours. The number of mold and yeast was countered after the cultivation on potato dextrose agar culture medium at 30 °C for 3 days. The effective preserving days was defined as the number of days when the viable count of microorganism, mold and yeast increased up to 10⁶/g.

### 3 RESULTS

| Test Group | effective preserving days |
|---|---|
| containing no chitosan | 2 |
| containing deoxydizing agent alone | 3 |
| containing chitosan 100 ppm alone | 3 |
| containing chitosan 250 ppm alone | 5 |
| containing chitosan (100 ppm) with deoxydizing agent | 10 |

### THE EFFECT OF THE INVENTION

According to the present invention a remarkable preservative effect can be obtained against the deterioration of foods occurred by yeast or microorganism, which cannot be effectively prevented by a single use of deoxydizing agent or of a natural antimicrobial agent such as protamine, chitosan, lysozyme and the like. Such a preservation can be effected using the natural antimicrobial agents specified even in the amount of less than half in case of single use thereof.

## Claims

1. A method of preserving food, comprising adding to food at least one kind of a natural antimicrobial agent selected from the group consisting of protamin, chitosan and lysozyme, and storing the food in the presence of a deoxidizing agent, wherein the food containing said natural antimicrobial agent is sealed together with the deoxidizing agent in the same sealed package.

2. A method of preserving food according to claim 1, wherein the deoxidizing agent is an iron type deoxidizing agent.

3. A method of preserving food according to claim 1 or claim 2, wherein the natural antimicrobial agent is protamine, which is used in the amount of 10 - 50,000 ppm.

4. A method of preserving food according to claim 1 or claim 2, wherein the natural antimicrobial agent is chitosan, which is used in the amount of 5 - 50,000 ppm.

5. A method of preserving food according to claim 1 or claim 2, wherein the natural antimicrobial agent is lysozyme, which is used in the amount of 1 - 10,000 ppm.

6. A method preserving food according to claim 1, wherein the food is stored under circumstances of oxygen concentration of not more than 0.05% in the presence of the deoxidizing agent.

7. A method of preserving food according to any preceding claim, wherein the food is preserved from deterioration by action of yeast.

8. A method of preserving food according to any one of claims 1 to 6, wherein the food is preserved from deterioration by action of lactic acid bacteria.

9. A method of preserving food according to claim 8, wherein the lactic acid bacteria is from a group consisting of lactobacillus, lenconosstoc and streptococcus.

## Patentansprüche

1. Verfahren zur Konservierung von einem Nahrungsmittel, umfassend die Zugabe zu dem Nahrungsmittel von mindestens einer Art eines natürlichen antimikrobiellen Mittels, ausgewählt aus der Gruppe, bestehend aus Protamin, Chitosan und Lysozym, und Lagerung des Nahrungsmittels in Anwesenheit eines Desoxidationsmittels, wobei das Nahrungsmittel, welches das natürliche antimikrobielle Mittel enthält, zusammen mit dem Desoxidationsmittel in der gleichen versiegelten Packung versiegelt ist.

2. Verfahren zur Konservierung von einem Nahrungsmittel nach Anspruch 1, wobei das Desoxidationsmittel ein Desoxidationsmittel des Eisentyps ist.

3. Verfahren zur Konservierung von einem Nahrungsmittel nach Anspruch 1 oder 2, wobei das natürliche antimikrobielle Mittel Protamin ist, welches in einer Menge von 10 bis 50.000 ppm verwendet wird.

4. Verfahren zur Konservierung von einem Nahrungsmittel nach Anspruch 1 oder 2, wobei das natürliche antimikrobielle Mittel Chitosan ist, welches in einer Menge von 5 bis 50.000 ppm verwendet wird.

5. Verfahren zur Konservierung von einem Nahrungsmittel nach Anspruch 1 oder 2, wobei das natürliche antimikrobielle Mittel Lysozym ist, welches in einer Menge von 1 bis 10.000 ppm verwendet wird.

6. Verfahren zur Konservierung von einem Nahrungsmittel nach Anspruch 1, wobei das Nahrungsmittel bei solchen Bedingungen gelagert wird, daß die Sauerstoffkonzentration nicht mehr als 0,05% in Anwesenheit des Desoxidationsmittels beträgt.

7. Verfahren zur Konservierung von einem Nahrungsmittel nach einem der vorhergehenden Ansprüche, wobei das Nahrungsmittel vor der Zersetzung durch den Einfluß von Hefe konserviert ist.

8. Verfahren zur Konservierung von einem Nahrungsmittel nach einem der Ansprüche 1 bis 6, wobei das Nahrungsmittel vor der Zersetzung durch Einwirkung von Milchsäurebaktieren konserviert ist.

9. Verfahren zur Konservierung von Nahrungsmitteln nach Anspruch 8, wobei die Milchsäurebakterien aus der Gruppe, bestehend aus Lactobacillus, Lenconosstoc und Streptococcus, stammen.

## Revendications

1. Procédé de conservation d'aliments comprenant les opérations qui consistent à ajouter aux aliments au moins un type d'agent antimicrobien naturel choisi dans le groupe constitué de protamine, chitosan et lysozyme et à conserver les aliments en présence d'un agent réducteur, dans lequel on enferme hermétiquement les aliments contenant ledit agent antimicrobien naturel en association avec l'agent réducteur dans le même emballage hermétique.

2. Procédé de conservation des aliments selon la revendication 1, dans lequel l'agent réducteur est un agent réducteur du type fer.

3. Procédé de conservation d'aliments selon la revendication 1 ou 2, dans lequel l'agent antimicrobien naturel est la protamine que l'on utilise en une quantité comprise entre 10 et 50000 ppm.

4. Procédé de conservation d'aliments selon la revendication 1 ou 2, dans lequel l'agent antimicrobien naturel est le chitosan que l'on utilise en une quantité comprise entre 5 et 50000 ppm.

5. Procédé de conservation d'aliments selon la revendication 1 ou 2, dans lequel l'agent antimicrobien naturel est le lysozyme que l'on utilise en une quantité comprise entre 1 et 10000 ppm.

6. Procédé de conservation d'aliments selon la revendication 1, dans lequel on conserve les aliments dans des conditions de concentration en oxygène ne dépassant pas 0,05% en présence de l'agent réducteur.

7. Procédé de conservation d'aliments selon l'une quelconque des revendications précédentes, dans lequel on protège les aliments d'une détérioration par l'action de levures.

8. Procédé de conservation d'aliments selon l'une quelconque des revendications 1 à 6, dans lequel on protège les aliments d'une détérioration par l'action de bactéries issues de l'acide lactique.

9. Procédé de conservation d'aliments selon la revendication 8, dans lequel les bactéries issues de l'acide lactique proviennent d'un groupe constitué de lactobacilles, leuconostoques et streptocoques.
